(19) **Europäisches Patentamt European Patent Office Office européen des brevets**

(11) **EP 3 815 600 A1**

(12) **EUROPEAN PATENT APPLICATION**

(43) Date of publication:
05.05.2021 Bulletin 2021/18

(51) Int Cl.:
*A61B 5/00* (2006.01)      *A61B 5/02* (2006.01)

(21) Application number: 19205692.7

(22) Date of filing: 28.10.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• MUELLER, Manfred
5656 AE Eindhoven (NL)
• BABIC, Drazenko
5656 AE Eindhoven (NL)
• LUCASSEN, Gerhardus Wilhelmus
5656 AE Eindhoven (NL)
• KURANE, Aditee
5656 AE Eindhoven (NL)

(74) Representative: Philips Intellectual Property & Standards
High Tech Campus 5
5656 AE Eindhoven (NL)

(54) **INTRAVASCULAR OPTICAL DEVICE**

(57) The present invention relates to an intravascular device (10). The device comprises an elongate member (20), an optical fiber (30), and at least one optical interaction element (40). At least a part of the elongate member is configured to be inserted into a part of a vascular system of a patient. At least a part of the optical fiber is located within the elongate member. The optical fiber is configured to transmit optical wavelength radiation. The intravascular device is configured to emit optical wavelength radiation out of the elongate member in at least two optical radiation beams for being scattered and/or reflected by a portion of the vascular system. The emission of the at least two optical radiation beams comprises interaction of the transmitted optical wavelength radiation with the at least one optical interaction element. The intravascular device is configured to collect at least some of the scattered and/or reflected optical wavelength radiation and couple the at least some of the scattered and/or reflected optical wavelength radiation into the optical fiber comprising utilization of the at least one optical interaction element.

FIG. 12

**Description**

FIELD OF THE INVENTION

The invention relates to an intravascular device, an intravascular microcatheter and guidewire device, an intravascular investigation system, a method of intravascular investigation with an intravascular investigation system and to a computer program element. The intravascular device may for example be a guidewire, a catheter or a microcatheter.

BACKGROUND OF THE INVENTION

The general background of the invention is blood clots, and in particular the provision of information to support its treatment, for example by thrombolysis or thrombectomy. Ischemic stroke is a common cause of death in the developed world and the leading cause of acquired neurological disability. In high-income countries, a substantial increase in the number of individuals affected by stroke is expected due to increasing life expectancy.

Thrombectomy, i.e. the physical removal of a blood clot, has been shown to be superior to thrombolysis in treating acute strokes. This has led to the development of various thrombectomy devices. Currently-available devices include stent-retrieval devices like the Embotrap from Johnson & Johnson, the Trevo ProVue from Stryker, the Solitaire from Covidien, and the Penumbra series of aspiration thrombectomy devices from Penumbra. Achieving first-time-right treatment is critical in thrombectomy because the time window available for treatment is short. Choosing the wrong treatment device can necessitate additional attempts to remove the blood clot, thereby lengthening the procedure time. Each thrombectomy attempt can take between five and ten minutes. Choosing the wrong device, and thereby necessitating the use of a subsequent different may therefore lead to an increase in medical complications as well as increased treatment cost.

A complicating factor for thrombectomy is that blood clots have different compositions. These pose different risks during thrombectomy: see for example T. Andersson, "The importance of clot properties in endovascular stroke therapy", https://neuronewsinternational.com/the-importance-of-clot-properties-in-endovascular-stroke-therapy/ (2015). The issues include the following: i) clots that are rich in red blood cells may be fragile and have a risk of clot break-up ii) clots that are rich in Fibrin may have a consistency that makes them difficult to grasp with a thrombectomy device; iii) about 15% of clots resist thrombectomy.

The ability to determine to determine which treatment device to use to treat a blood clot would also be advantageous for peripheral venous clots. The composition of peripheral venous clots differs from clots that may lead to ischemic stroke. If left untreated, or if treated with an incorrect treatment device, a mobilized peripheral venous blood clot may for example be transported to the lung and trigger further medical complications.

Thus, for both peripheral venous clots as well as clots that may trigger stroke, it would therefore be advantageous for a physician to know the composition of a blood clot in advance of selecting a thrombectomy device. Thus, there is a need to address these and other related issues.

SUMMARY OF THE INVENTION

It would be advantageous to have improved instrumentation to support intravascular investigations.

The object of the present invention is solved with the subject matter of the independent claims, wherein further embodiments are incorporated in the dependent claims. It should be noted that the following described aspects and examples of the invention apply to the intravascular devices, to the intravascular microcatheter and guidewire device, to the intravascular investigation system, and to the method of intravascular investigation, and to the computer program element and to the computer readable medium.

According to a first aspect, there is provided an intravascular device, comprising:

- an elongate member;
- an optical fiber; and
- at least one optical interaction element;

At least a part of the elongate member is configured to be inserted into a part of a vascular system of a patient. At least a part of the optical fiber is located within the elongate member. The optical fiber is configured to transmit optical wavelength radiation. The intravascular device is configured to emit optical wavelength radiation out of the elongate member in at least two optical radiation beams for being scattered and/or reflected by a portion of the vascular system. The emission of the at least two optical radiation beams comprises interaction of the transmitted optical wavelength radiation with the at least one optical interaction element. The intravascular device is configured to collect at least some of the scattered and/or reflected optical wavelength radiation and couple the at least some of the scattered and/or reflected optical wavelength radiation into the optical fiber comprising utilization of the at least one optical interaction element.

The intravascular device may be used with an optical radiation source that generates broadband optical radiation. The broadband optical radiation may be provided either simultaneously by means of a broadband optical source, or by scanning a narrowband filter across an output of a broadband optical source, or by scanning the wavelength of a monochromatic optical radiation source across multiple wavelengths such that broadband optical radiation is coupled into and transmitted by the optical fiber. The optical radiation then exits the intravascular device and interacts with the vasculature of the patient. Reflected and/or scattered optical radiation from for example a blood clot can be presented to a detection unit such as an optical detector or a spectrometer. By arranging that at least two optical radiation beams are emitted from the interventional device, different regions of the vascular structure may be interrogated with the interventional device in a fixed position. Moreover, the at least two optical radiation beams may optionally interrogate their corresponding regions with different optical wavelengths. In one implementation, a first, forward-looking optical radiation beam may be provided that emits optical wavelength radiation in an axial direction with respect to a distal end of the interventional device, and a second, side-looking optical radiation beam may be provided that emits or projects optical wavelength radiation radially outwards with respect to a longitudinal axis of the interventional device. In so doing, as the interventional device is advanced in the vasculature, the first optical beam may be used to characterize the vasculature prior to the second optical beam. The second optical beam may provide improved optical measurements due to improved optical contact between the blood clot and the interventional device as the interventional device is moved past the blood clot. Additionally, by providing two optical radiation beams, with for example one extending out of the end of the interventional device and a second extending out of a side wall of the interventional device, a physician can bend the end of the interventional device, which may be a guidewire, as required in order to probe regions as required. Then, a second optical radiation beam can extend out of the side wall of the interventional device at a distance from the end, where the interventional device is not bent, and provide for accurate measurements as the interventional device slides past the object being interrogated. Two or more optical radiations beams may be emitted out of the side wall of the interventional device, or indeed two or more optical radiation beams could be projected out of the front of the interventional device. These beams could overlap, but comprise different wavelength ranges. The different wavelength ranges could be provided through appropriate scanning or switching or could have different angular directions, and have the same wavelength range or different wavelength ranges.

In an example, the at least two optical radiation beams comprises a first optical radiation beam emitted out of a side wall of the elongate member.

In an example, the at least two optical radiation beams comprises a second optical radiation beam emitted out of the side wall of the elongate member.

In an example, a wavelength range of the first optical radiation beam is different to a wavelength range of the second optical radiation beam.

In this manner, different optical wavelengths can be emitted and as such different volumes can be optically probed.

In an example, the first optical radiation beam is emitted out of the elongate member at a first longitudinal position of the elongate member and the second optical radiation beam is emitted out of the elongate member at a second longitudinal position of the elongate member different to the first longitudinal position.

Thus, for example there can be one optical radiation beam emitted sideways from the intravascular device having one wavelength range and a second optical radiation beam can be parallel to that first beam but have a second wavelength range and be emitted at a different position along the length of the intravascular device, such as a guidewire. Then as the device is moved past a blood clot the blood clot is interrogated over one wavelength range and then interrogated over a second wavelength range, and the physician does not have to rotate the device, but merely has to move it longitudinally. In other words, one optical radiation beam can be emitted sideways at for example 4 centimeters from the tip of the intravascular device having one wavelength range and a second beam can be emitted at for example 5 centimeters from the tip of the intravascular device in the same direction and parallel to the first optical radiation beam, but have a different wavelength range,

In an example, the at least two optical radiation beams comprises a optical radiation beam emitted out of an end wall of the elongate member.

Thus, a forward directed optical radiation beam can be provided.

In an example, a wavelength range of the first optical radiation beam is different to a wavelength range of the optical radiation beam emitted out of the end wall of the elongate member.

In an example, a wavelength range of the second optical radiation beam is different to the wavelength range of the optical radiation beam emitted out of the end of the elongate member.

In an example, the at least one optical interaction element comprises a wavelength selective element.

In an example, a portion of the optical fiber at a distal end of the optical fiber is fixedly connected to the elongate member. The at least a part of the optical fiber located within the elongate member other than the fixed distal end is not fixedly connected to the elongate member.

According to a second aspect, there is provided an intravascular device, comprising:

- an elongate member;
- an optical fiber, and
- at least one optical interaction element.

At least a part of the elongate member is configured to be inserted into a part of a vascular system of a patient. At least a part of the optical fiber is located within the elongate member. The optical fiber is configured to transmit optical wavelength radiation. The intravascular device is configured to emit optical wavelength radiation out of the elongate member in an optical radiation beam that forms an annular emission profile substantially perpendicular to a longitudinal axis of the elongate member for being scattered and/or reflected by a portion of the vascular system. The emission of the optical radiation beam comprises interaction of the transmitted optical wavelength radiation with the at least one optical interaction element. The intravascular device is configured to collect at least some of the scattered and/or reflected optical wavelength radiation and couple the at least some of the scattered and/or reflected optical wavelength radiation into the optical fiber comprising utilization of the at least one optical interaction element.

Thus, the intravascular device emits and collects optical wavelength radiation to/from different angles around the elongated member at the same longitudinal location or position. In this manner, the interventionist does not need to torque or rotate the guidewire at the longitudinal position in order to interrogate a blood clot, because the optical wavelength radiation is emitted all around the intravascular device at that location.

According to a third aspect, there is provided an intravascular microcatheter and guidewire device, comprising:

- a microcatheter; and
- an intravascular device according to the first aspect or an intravascular device according to the second aspect.

At least a part of the microcatheter is configured to be inserted into the part of the vascular system of the patient. The microcatheter comprises at least one optically-transmitting wall portion. The intravascular device is configured to slide within the microcatheter along a longitudinal axis of the microcatheter. The microcatheter and intravascular device are configured such that when the intravascular guidewire is positioned at one or more longitudinal positions along the longitudinal axis of the microcatheter, optical wavelength radiation is emitted out of the microcatheter through the at least one optically-transmitting wall portion of the microcatheter and scattered and/or reflected optical wavelength radiation enters the microcatheter through the at least one optically-transmitting wall portion of the microcatheter.

According to a fourth aspect, there is provided an intravascular investigation system, comprising:

- an intravascular device according to the first aspect, or an intravascular device according to the second aspect, or an intravascular microcatheter and guidewire device according to the third aspect;
- a optical radiation source;
- a optical radiation detector; and
- a processing unit.

The optical radiation source is configured to generate optical wavelength radiation over a broadband range and couple it into the optical fiber. The optical radiation detector is configured to generate at least one detection signal on the basis of the scattered and/or reflected optical wavelength radiation. The processing unit is configured to determine at least one spectrally resolved data set on the basis of the at least one detection signal. The processing unit is configured to determine information about a blood clot on the basis of the at least one spectrally resolved data set.

In this manner optical spectroscopy is used to provide information relating to a suspected occluding structure, such as blood clots, and the location of the suspected occluding structure can be determined. Through having more than one optical radiation beam being used to probe the vascular structure, the location of the blood clot and its characteristics can be determined more efficiently and effectively. Thus, a determination can be made if a blood clot is present, and if a blood clot is present it is possible to discriminate between different types of blood clots, for example to determine if a blood clot is rich in red blood cells, to determine if a blood clot is rich in Fibrin, and to determine if a blood clot is of a type that resists thrombectomy and will have to be treated by thrombolysis.

In this other words, the correct thrombectomy device to remove the blood clot can be determined, thereby decreasing treatment time, reducing cost, and reducing patient risk by reducing the need to undertake a second blood clot removal procedure if the incorrect type of device was initially chosen.

According to a fifth aspect, there is provided a method of intravascular investigation with an intravascular investigation system according to the fourth aspect wherein the method comprises:

- generating optical wavelength radiation over a broadband range with the optical radiation source;
- coupling the broadband optical wavelength radiation into the optical fiber of the intravascular device or the intravascular microcatheter and guidewire device;
- collecting scattered and/or reflected optical wavelength radiation from a vascular structure of the patient with the intravascular device or the intravascular microcatheter and guidewire device;
- generating by the optical radiation detector at least one detection signal on the basis of the scattered and/or reflected optical wavelength radiation;
- determining by the processing unit at least one spectrally resolved data set on the basis of the at least one detection signal; and
- determining by the processing unit information about a blood clot on the basis of the at least one spectrally resolved data set.

According to another aspect, there is provided a computer program element controlling a device and/or system as previously described which, if the computer program element is executed by a processing unit, is adapted to perform the method steps as previously described.

According to another aspect, there is provided a computer readable medium having stored computer element as previously described.

According to a sixth aspect, there is provided a system, a corresponding method for determining the composition of a peripheral venous clot with the system, and a corresponding computer program product.

The system for determining the composition of a peripheral venous clot includes:

an intravascular device for determining blood clot composition in the peripheral vasculature comprising:

- an elongate member; and
- an optical fiber;

wherein at least a part of the elongate member is configured to be inserted into a part of a vascular system of a patient;
wherein at least a part of the optical fiber is located within the elongate member;
wherein, the optical fiber is configured to transmit optical wavelength radiation;
wherein the intravascular device is configured to emit a portion of the optical wavelength radiation out of the elongate member for being scattered and/or reflected by a portion of the vascular system;
wherein the intravascular device is configured to collect at least some of the scattered and/or reflected optical wavelength radiation and to couple the at least some of the scattered and/or reflected optical wavelength radiation into the optical fiber; and the system further includes:

an optical radiation source;
an optical radiation detector;
and a processing unit;
wherein the optical radiation source is configured to generate optical wavelength radiation over a broadband range and couple it into the optical fiber;
wherein the optical radiation detector is configured to generate at least one detection signal on the basis of the scattered and/or reflected optical wavelength radiation;
wherein the processing unit is configured to determine at least one spectrally resolved data set on the basis of the at least one detection signal;
wherein the at least one spectrally resolved data set comprises a spectrum corresponding to collagen; and
wherein the processing unit is configured to determine a collagen content from the spectrum corresponding to collagen and to determine information about a blood clot on the basis of the collagen content.

The corresponding method of determining the collagen content of a peripheral vascular clot with the aforementioned intravascular investigation system for use in determining blood clot composition in the peripheral vasculature may include the steps of:

- generating optical wavelength radiation over a broadband range with the optical radiation source;
- coupling the broadband optical wavelength radiation into the optical fiber of the intravascular device or the intravascular microcatheter and guidewire device;
- collecting scattered and/or reflected optical wavelength radiation from a vascular structure of the patient with the intravascular device or the intravascular microcatheter and guidewire device;
- generating by the optical wavelength radiation detector at least one detection signal on the basis of the scattered and/or reflected optical wavelength radiation;
- determining by the processing unit at least one spectrally resolved data set on the basis of the at least one detection signal, the at least one spectrally resolved data set comprising a spectrum corresponding to collagen; and
- determining by the processing unit a collagen content from the spectrum corresponding to collagen, and information about a blood clot on the basis of the collagen content.

A corresponding computer program product comprising instructions which when executed by a processor causes the processor to execute the method is also provided.

Advantageously, the benefits provided by any of the above aspects equally apply to all of the other aspects and vice versa.

The above aspects and examples will become apparent from and be elucidated with reference to the embodiments described hereinafter.

BRIEF DESCRIPTION OF THE DRAWINGS

Exemplary embodiments will be described in the following with reference to the following drawings:

Fig. 1 shows a schematic example of an intravascular device;
Fig. 2 shows a schematic example of an intravascular microcatheter and guidewire device, an intravascular investigation system;
Fig. 3 shows a method of intravascular investigation;
Fig. 4 shows a Diffuse Reflectance Spectroscopy (DRS) system for blood clot discrimination;
Fig. 5 shows a state-of-the art tissue sensing guidewire with the sensing happening at the tip of the guidewire;

Fig. 6 shows an example of an intravascular guidewire;

Fig. 7 shows an example of an intravascular guidewire;

Fig. 8 shows an example of an intravascular microcatheter and guidewire device;

Fig. 9 shows an example of an intravascular guidewire;

Fig. 10 shows an example of an intravascular guidewire;

Fig. 11 shows an example of an intravascular guidewire;

Fig. 12 shows an example of an intravascular guidewire;

Fig. 13 shows an example of an intravascular guidewire;

Fig. 14 illustrates the variation in measured light intensity (arbitrary units) versus wavelength in nanometers for three blood clot analogue samples; and

Fig. 15 illustrates the predictive ability (Predictor) versus Collagen fraction (%) for several measured blood clot analogue samples.

DETAILED DESCRIPTION OF EMBODIMENTS

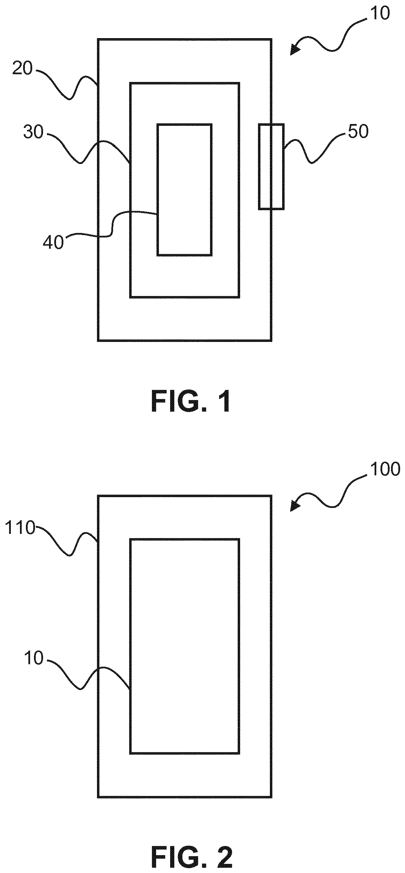

Fig. 1 shows an example of an intravascular device 10. The device comprises an elongate member 20, an optical fiber 30, and at least one optical interaction element 40. At least a part of the elongate member is configured to be inserted into a part of a vascular system of a patient. At least a part of the optical fiber is located within the elongate member. The optical fiber is configured to transmit optical wavelength radiation. The intravascular device is configured to emit optical wavelength radiation out of the elongate member in at least two optical radiation beams for being scattered and/or reflected by a portion of the vascular system. The emission of the at least two optical radiation beams comprises interaction of the transmitted optical wavelength radiation with the at least one optical interaction element. The intravascular device is configured to collect at least some of the scattered and/or reflected optical wavelength radiation and couple the at least some of the scattered and/or reflected optical wavelength radiation into the optical fiber comprising utilization of the at least one optical interaction element.

In an example, the optical wavelength radiation is generated by an optical radiation source that emits broadband optical radiation simultaneously. In an example, the optical wavelength radiation is generated by an optical radiation source that emits narrowband optical radiation and scans the emitted optical wavelengths over a wavelength range to generate broadband optical radiation that is transmitted by the optical fiber.

In an example, the at least one optical interaction element comprises at least one optically-transmitting wall portion 50 of the elongate member, and emitted optical wavelength radiation is directed out of the elongate member through the at least one optically-transmitting wall portion and a portion of the scattered and/or reflected optical wavelength radiation enters back through the at least one optically-transmitting wall portion. Thus, the window can protect the optical fiber, and can be a wavelength sensitive filter, thus providing a convenient manner to select probing wavelength ranges, and where exit windows for different beams can have different passband wavelength ranges, providing an efficient manner to provide beams having different wavelength ranges. These windows can be at side walls of the elongate member, and a window can be at the end wall of the elongate member if required.

In an example, the intravascular device is a guidewire.

In an example, the intravascular device is a microcatheter.

In an example, the elongate member comprises a radio opaque marker.

In an example, the elongate member is a tube.

In an example, the optical fiber comprises a radio opaque marker.

In this manner, an operator can determine in what specific direction or directions optical wavelength radiation will be emitted in order to maximize the overlap between a blood clot being examined and the spectroscopic sensing volume.

According to an example, the at least two optical radiation beams comprises a first optical radiation beam emitted out of a side wall of the elongate member.

In an example, the first optical radiation beam is emitted out of the side wall of the elongate member at least 3 centimeters from an end of the elongate member.

In an example, the first optical radiation beam is emitted in a direction perpendicular to a longitudinal axis of the elongate member.

In an example, the at least one optical interaction element comprises a beam splitter.

In an example, the at least one optical interaction element comprises a 45 degree beam splitter.

In an example, the at least one optical interaction element comprises a region of the optical fiber exhibiting total internal reflection.

In an example, the at least one optical interaction element comprises an optical wavelength filter.

In an example, the at least one optical interaction element comprises a Bragg fiber grating.

According to an example, the at least two optical radiation beams comprises a second optical radiation beam emitted out of the side wall of the elongate member.

In an example, the second optical radiation beam is emitted out of the side wall of the elongate member at least 3 centimeters from an end of the elongate member.

In an example, the second optical radiation beam is emitted in a direction perpendicular to the longitudinal axis of the elongate member.

In an example, the at least one optical interaction element comprises a beam splitter.

In an example, the at least one optical interaction element comprises a 45 degree beam splitter.

In an example, the at least one optical interaction element comprises a Bragg fiber grating.

In an example, the at least one optical interaction element comprises a wavelength passband filter.

In an example, the at least one optical interaction element comprises a wavelength selective window.

According to an example, a wavelength range of the first optical radiation beam is different to a wavelength range of the second optical radiation beam.

According to an example, the first optical radiation beam is emitted out of the elongate member at a first longitudinal position of the elongate member and the second optical radiation beam is emitted out of the elongate member at a second longitudinal position of the elongate member different to the first longitudinal position.

According to an example, the at least two optical radiation beams comprises a optical radiation beam emitted out of an end wall of the elongate member.

In an example, the optical radiation beam emitted out of the end wall of the elongate member is emitted in a direction parallel to the longitudinal axis of the elongate member.

According to an example, a wavelength range of the first optical radiation beam is different to a wavelength range of the optical radiation beam emitted out of the end wall of the elongate member.

According to an example, a wavelength range of the second optical radiation beam is different to the wavelength range of the optical radiation beam emitted out of the end of the elongate member.

According to an example, the at least one optical interaction element comprises a wavelength selective element.

In an example, the at least one wavelength selective element comprises a Bragg fiber grating.

In an example, the at least one wavelength selective element comprises a wavelength passband filter.

In an example, the at least one wavelength selective element comprises a wavelength selective window. Thus, a wavelength selective window can be placed at a or in the wall of the elongate member, at the side wall and/or the front wall and provides an efficient manner to provide for optical wavelength radiation that is used to interrogate material via optical wavelength radiation that is transmitted through that element only to have a certain specific wavelength range.

According to an example, a portion of the optical fiber at a distal end of the optical fiber is fixedly connected to the elongate member. The at least a part of the optical fiber located within the elongate member other than the fixed distal end is not fixedly connected to the elongate member.

Fig. 1 can also represent another example of an intravascular device 10. This intravascular device 10 comprises an elongate member 20, an optical fiber 30, and at least one optical interaction element 40. At least a part of the elongate member is configured to be inserted into a part of a vascular system of a patient. At least a part of the optical fiber is located within the elongate member. The optical fiber is configured to transmit optical wavelength radiation. The intravascular device is configured to emit optical wavelength radiation out of the elongate member in an optical radiation beam that forms an annular emission profile substantially perpendicular to a longitudinal axis of the elongate member for being scattered and/or reflected by a portion of the vascular system. The emission of the optical radiation beam comprises interaction of the transmitted optical wavelength radiation with the at least one optical interaction element. The intravascular device is configured to collect at least some of the scattered and/or reflected optical wavelength radiation and couple the at least some of the scattered and/or reflected optical wavelength radiation into the optical fiber comprising utilization of the at least one optical interaction element.

In an example, the annular optical wavelength radiation emission is emitted out of the side wall of the elongate member at least 3 centimeters from an end of the elongate member.

Thus, an interventionist can bend the tip of the guidewire as required to provide for example a probing beam out of the end of the guidewire at a correct angle, without affecting the lateral transmission beam or beams.

In an example, one or more optical interaction element of the at least one optical interaction element is rotationally symmetric about a longitudinal axis of the elongate member.

In an example, the one or more of the at least one optical interaction element that is rotationally symmetric comprises a conical structure.

In an example, the at least one optical interaction element comprises at least one optically-transmitting wall portion 50 of the elongate member, and emitted optical wavelength radiation is directed out of the elongate member through the at least one optically-transmitting wall portion and a portion of the scattered and/or reflected optical wavelength radiation enters back through the at least one optically-transmitting wall portion. Thus, the window can protect the optical fiber, and can be a wavelength sensitive filter, thus providing a convenient manner to select probing wavelength ranges, and where exit windows for different beams can have different passband wavelength ranges, providing an efficient manner to provide beams having different wavelength ranges. These windows can be at side walls of the elongate member, and a window can be at the end wall of the elongate member if required.

In an example, the at least one optical interaction element is configured to emit a further optical radiation beam sideways out of the elongate member at a different longitudinal position to the annular emission profile. The optical radiation beam can have a smaller angular spread than the annular emission profile. Thus, the annular emission profile (or doughnut shaped emission) probing can be used to quickly find the location of a clot, then the second optical radiation beam can be moved to that location, and through rotation of the guidewire the optical radiation beam can be used to interrogate the blood clot with a high signal to noise because the signal can be limited to the blood clot and not have background vascular system information.

Fig. 2 shows an example of an intravascular microcatheter and guidewire device 100 comprising a microcatheter 110, and an intravascular device 10 as described with respect to either of the two embodiments described with respect to Fig. 1. At least a part of the microcatheter is configured to be inserted into the part of the vascular system of the patient. The microcatheter comprises at least one optically-transmitting wall portion. The intravascular device is configured to slide within the microcatheter along a longitudinal axis of the microcatheter. The microcatheter and intravascular device are configured such that when the intravascular guidewire is positioned at one or more longitudinal positions along the longitudinal axis of the microcatheter, optical wavelength radiation is emitted out of the microcatheter through the at least one optically-transmitting wall portion of the microcatheter and scattered and/or reflected optical wavelength radiation enters the microcatheter through the at least one optically-transmitting wall portion of the microcatheter.

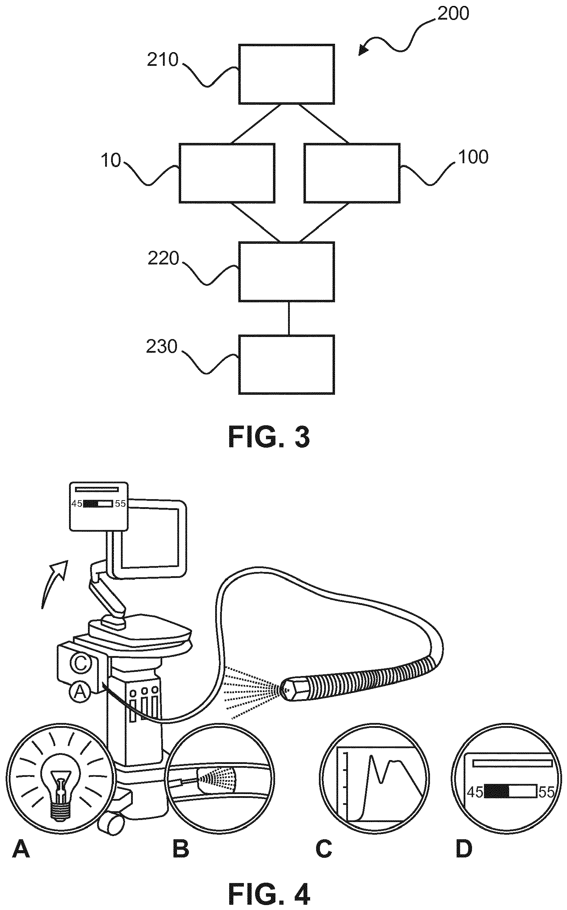

Fig. 3 shows an example of an intravascular investigation system 200. The system comprises an intravascular device 10 as described with respect to either of the two embodiments described with respect to Fig. 1, or an intravascular device 100 according to claim 11, or an intravascular microcatheter and guidewire device 100 as described with respect to Fig. 2. The system 200 also comprises an optical radiation source 210, an optical radiation detector 220, and a processing unit 230. The optical radiation source is configured to generate optical wavelength radiation over a broadband range and couple it into the optical fiber. The optical radiation detector is configured to generate at least one detection signal on the basis of the scattered and/or reflected optical wavelength radiation. The processing unit is configured to determine at least one spectrally resolved data set on the basis of the at least one detection signal. The processing unit is configured to determine information about a blood clot on the basis of the at least one spectrally resolved data set.

In an example, the optical radiation source is configured to scan a narrowband or monochromatic optical wavelength radiation over a broadband and the at least one optical radiation signal comprises scattered and/or reflected optical radiation for each wavelength step of that scanning.

In an example, the optical radiation source is configured to provide broadband optical wavelength radiation in one beam, and the detector and processing unit can be part of a spectrometer that determines a "one shot" detection signal and spectrally resolved data set.

In an example, the processing unit is configured to provide an output indicating that spectra from vessel walls are detected.

In this way, a feedback loop is provided to the interventionist, who then knows when a blood clot is not being interrogated and can move the guidewire, either rotationally or longitudinally as required in order to interrogate the blood clot.

Associated with the system 200 is a method of intravascular investigation with an intravascular investigation system. The method comprises:

- generating optical wavelength radiation over a broadband range with the optical radiation source;
- coupling the broadband optical wavelength radiation into the optical fiber of the intravascular device or the intravascular microcatheter and guidewire device;
- collecting scattered and/or reflected optical wavelength radiation from a vascular structure of the patient with the intravascular device or the intravascular microcatheter and guidewire device;
- generating by the optical radiation detector at least one detection signal on the basis of the scattered and/or reflected optical wavelength radiation;
- determining by the processing unit at least one spectrally resolved data set on the basis of the at least one detection signal; and
- determining by the processing unit information about a blood clot on the basis of the at least one spectrally resolved data set.

The intravascular devices, intravascular microcatheter and guidewire device, intravascular investigation system, and method of intravascular investigation are now described in more detail with respect to specific embodiments, where reference is made to Figs. 4-13.

Fig. 4 shows a diffuse reflectance spectroscopy (DRS) system for blood clot discrimination. Investigations have shown that, amongst other optical analysis techniques, DRS can be used to discriminate between different types of blood clots and help the physician to make the best treatment decision. As shown at "A" an optical source emits optical wavelength radiation through an optical fiber. As shown at "B" optical wavelength radiation is scattered and absorbed in the blood clot and part of scattered optical wavelength radiation is received back into the optical fiber. As shown at "C" the optical wavelength radiation is received and analyzed by the spectrometer. As shown at "D", algorithms calculate physiological parameters, like red blood cell, fibrin and white blood cell content which are presented to the operator. Thus, in this system, optical wavelength radiation is used to illuminate a diffuse reflecting sample, which may for example be biological tissue. The new devices and system and method described herein can make use of an optical radiation source that scans a narrow emission band over a broad wavelength range to produce this optical wavelength radiation, such as by a scanning a wavelength-tunable laser, or can have an optical source that emits optical wavelength radiation across a plurality of optical wavelengths, or indeed it can consist of multiple narrowband optical sources (such as infrared or visible LEDs or lasers) that emit simultaneously or are caused to emit sequentially. Optical wavelength radiation is scattered and/ or absorbed by the sample. A part of the back-scattered optical wavelength radiation is collected and analyzed with an optical detector yielding a spectrum that is characteristic for the sample. Typically these spectra show a scattering background punctuated by characteristic dips caused by absorbers such as blood, water and fat. A detailed description of an example method of analyzing the data is described for example in the following publications: R. Nachabé, B. H. W. Hendriks, A. E. Desjardins, M. van der Voort, M. B. van der Mark, and H. J. C. M. Sterenborg, "Estimation of lipid and water concentrations in scattering media with diffuse optical spectroscopy from 900 to 1600 nm", J. Biomed. Opt. 15, (2010); Rami Nachabé, Benno H. W. Hendriks, Marjolein van der Voort, Adrien E. Desjardins, and Henricus J. C. M. Sterenborg, "Estimation of biological chromophores using diffuse optical spectroscopy: benefit of extending the UV-VIS wavelength range to include 1000 to 1600 nm", Optics Express 18 (2010) p1432; and R. Nachabé, et al., "Diagnosis of breast cancer using diffuse optical spectroscopy from 500 to 1600 nm: comparison of classification methods" J. Biomed. Opt. 16(8): p. 087010 (2011).

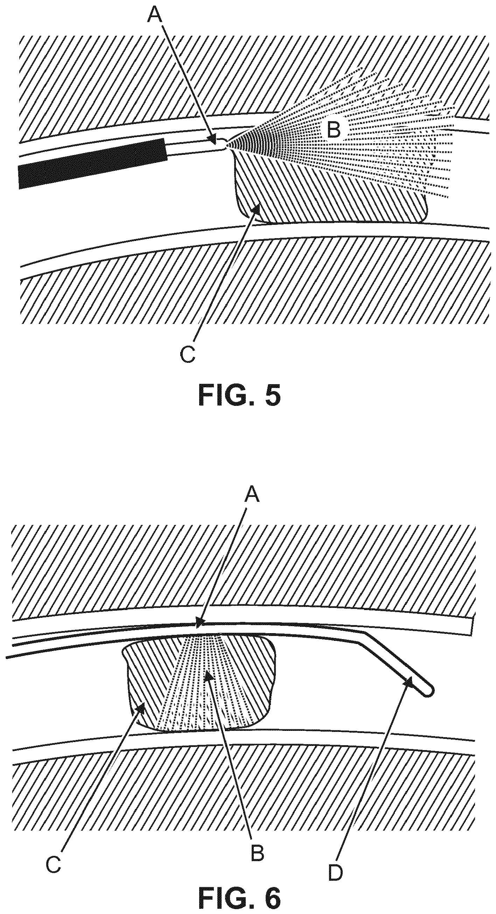

Fig. 5 shows a state-of-the art tissue-sensing guidewire disposed inside a blood vessel wherein optical sensing takes place at the tip of a guidewire. The blood vessel and blood vessel wall are illustrated, surrounding tissue being represented beyond the vessel wall. An "atraumatic, optical radiation-emitting tip" is represented by "A", a "sensing volume" by "B", and a "clot" by "C". Such systems are described for example in US5439000, US5601087A, US7532920B1, and US6445939B1. However, such sensing devices are sub-optimal for some of the sensing applications described above for a number of reasons, including: i) the tip of the guidewire is susceptible to sliding along the wall of the blood vessel, which might make it difficult to obtain good optical contact with the blood clot; ii) since the guidewire typically slides along the vessel wall, the sensed volume will include not only the blood clot but also the vessel wall and the surrounding tissue, which degrades the signal-to-noise-ratio and makes it more difficult to interpret the reflected optical wavelength radiation, iii) some interventionists may prefer to use guidewires with specially-shaped tip, this sometimes being a tip that they shape themselves, however such a tip can however interfere with optical radiation emission and collection at the tip; iv) neurological guidewires are typically designed to have a very floppy tip to avoid damage to the vasculature, whereas with a forward-sensing optical guidewire the necessary optical fiber will stiffen the tip, thereby limiting its floppiness; v) since blood clots can be inhomogeneous, physicians would like to scan along the length of blood clot without losing the guidewire's position, this being difficult when sensing occurs at the tip of the guidewire because the guidewire needs to be completely withdrawn from the clot to sense the proximal part of the clot; vi) for regulatory, training, or safety reasons it would be beneficial to have the option to measure clot composition from within a neurological catheter, such that the measuring guidewire does not need to come into direct contact with the vessel wall or the blood clot.

Thus, for a state-of-the art tissue sensing guidewire, with the sensing occurring at the tip of the guidewire as shown in Fig. 5, the sensing volume may be sub-optimal. Some of these hindrances are addressed by the devices, system and method described herein.

Fig. 6 shows an example of an intravascular guidewire. A "Side-emitting guidewire" is represented by "A", a "Sensing volume" by "B", a "clot" by "C", and an "arbitrarily-shaped tip" by "D". In this embodiment, the optical wavelength radiation is emitted and received at the shaft of the wire a distance from the tip (for example at least 3 centimeters away from the tip). The tip can thus be shaped, bent or otherwise designed as desired without influencing the spectroscopic sensing. Optical wavelength radiation is emitted and received substantially perpendicularly with respect to the main axis of the wire. The interventionist can then "pass the clot" but once this is accomplished, then may perform a pullback and scan the clot composition along its length without losing wire position. In order to get the best signal, the interventionist may have to torque (i.e. rotate) the wire to optimize the overlap between the sensing volume and the blood clot and to minimize the overlap between the sensing volume and the vessel wall, and potentially other surrounding tissue. In order to help find the correct rotational angle, the wire can incorporate a radiopaque marker indicating the sensing direction. Alternatively, feedback may be provided to an operator in the form of a warning when spectra corresponding to a vessel wall is detected.

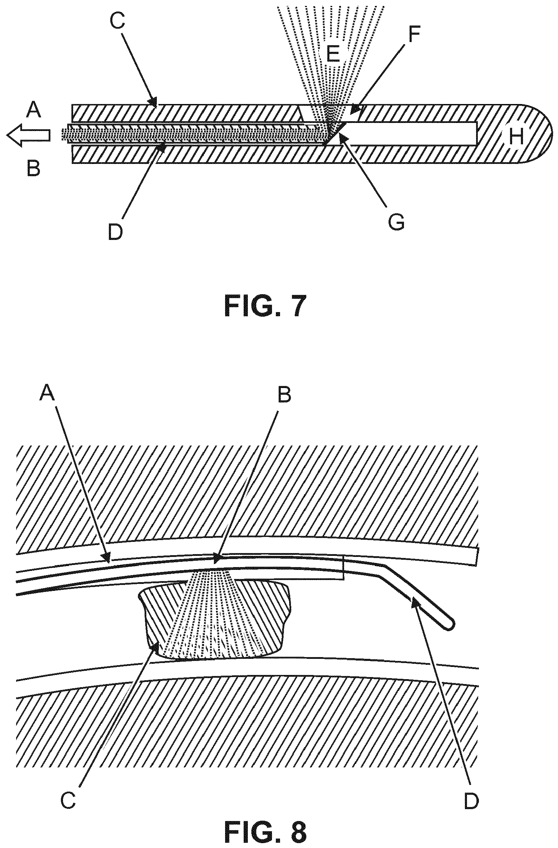

Fig. 7 shows an example of an intravascular guidewire. The arrow shows the direction towards an "optical radiation source" represented by "A" and a "spectrometer" represented by "B", which are outside of the intravascular part of the guidewire at the other end of the optical fiber. A "hollow tube" by "C", an "optical fiber" by "D", "emitted optical wavelength radiation" by "E", "hole with transparent filing" by "F", "cleavage with reflective coating" by "G", and a "tip" by "H". Typically, the wire will comprise a hollow tube or coil with an optical fiber in the inside of the lumen. A hole or gap in the tube at the distal end of the fiber allows optical wavelength radiation to exit and enter the lumen. The hole or gap will typically be sealed with a transparent material to ensure optimal optical contact with the outside and to prevent blood from entering. The fiber will typically only be fixed at its distal end to allow the fiber to slide inside the lumen to facilitate bending. Alternatively, the fiber can be placed in a grove along the side of the wire. The wire can be surrounded by additional layers (e.g. a shrink tube). These surrounding layers will also be transparent for the wavelengths for the wavelengths used for spectroscopy. Optical wavelength radiation is emitted from the fiber in a direction substantially perpendicular to the main axis of the guidewire (which is typically also the main axis of the fiber). Ideally the same fiber will be used to collect optical wavelength radiation reflected from the surrounding tissue and the surrounding blood. To deflect the optical wavelength radiation by approximately 90 degrees out/into the fiber, the end of the fiber can be cleaved at an angle of approximately 45 degrees. Optionally the cleaved edge of the fiber can be covered with a reflective coating. Alternatively, one can make use of a separate mirror. Optionally, the mirror or the facets of the fiber may be shaped such that the optical wavelength radiation is focused or de-focused.

Fig. 8 shows an example of an intravascular microcatheter and guidewire device. A "transparent catheter" is represented by "A", a "side emitting guidewire" by "B", a "Clot" by "C", and an "arbitrarily shaped tip" by "D". In this embodiment, the wire is combined with a microcatheter that is transparent for the wavelengths used in the spectroscopy at its distal end. The wire is inserted once the microcatheter has passed the clot and the spectroscopic measurement is taken through the walls of the microcatheter and without the wire leaving the microcatheter. A mechanical block can be used to prevent the tip of the wire from exiting the microcatheter. A benefit of this arrangement is that the requirements for the wire (and the wire operator) are lower than for embodiments without a transparent microcatheter, since the wire itself never comes into contact with the clot or the vessel wall.

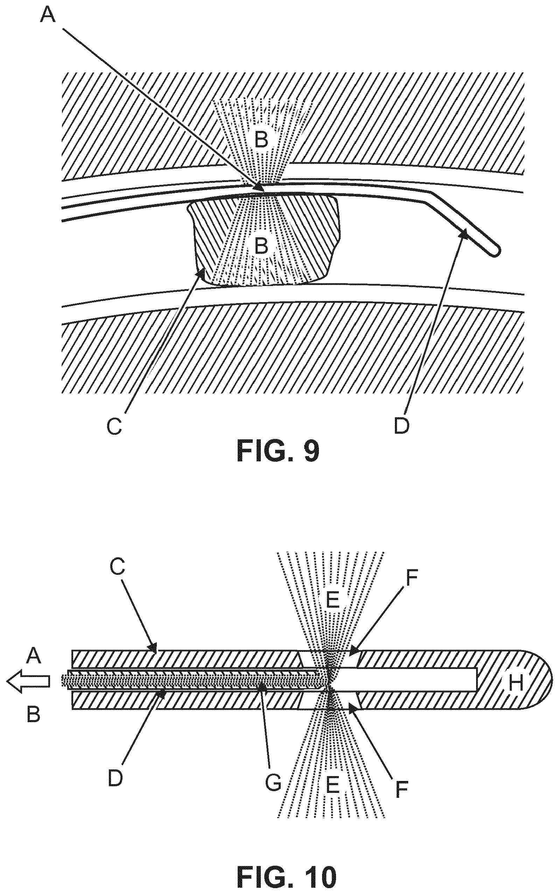

Fig. 9 shows an example of an intravascular guidewire. A "side emitting guidewire" is represented by "A", two "sensing volumes" by "B", a "clot" by "C", and an "arbitrarily shaped tip" by "D". In this embodiment, the neuro-interventionist does not have to rotate the wire. This is because in this embodiment the sensing volume covers a 360 degree ring around the wire. This ensures that the clot is always within the sensing volume of the wire. It is to be noted that the sensing volume will also contain the vessel wall and possibly surrounding tissue, leading to a lower signal-to-noise ratio than in embodiments where only the blood clot is interrogated. In this embodiment the optical fiber is inside a hollow tube (i.e. not in a groove). Instead of a single hole/window the tube has multiple openings or a single slit (with windows if required). To deflect the optical wavelength radiation in a 360 degree radius the tip of the fiber may have a conical shape (as shown in Fig. 10), which may for example be achieved by polishing. In Fig. 10 an "optical radiation source" is represented by "A", a "spectrometer" by "B", a "hollow tube" by "C", an "optical fiber" by "D", "emitted optical wavelength radiation" by "E", "holes or slit with transparent filing" by "F", "cone" by "G", and a "tip" by "H"). If the index of refraction of the fiber and the cone angle are suitable, total internal reflection at the cone will deflect the optical wavelength radiation from the fiber into an annular shape that expands laterally away from the intravascular device. Thus, a sensed volume can be a "doughnut"-shaped volume, where optical wavelength radiation from that volume can be scattered/reflected back and be collected.

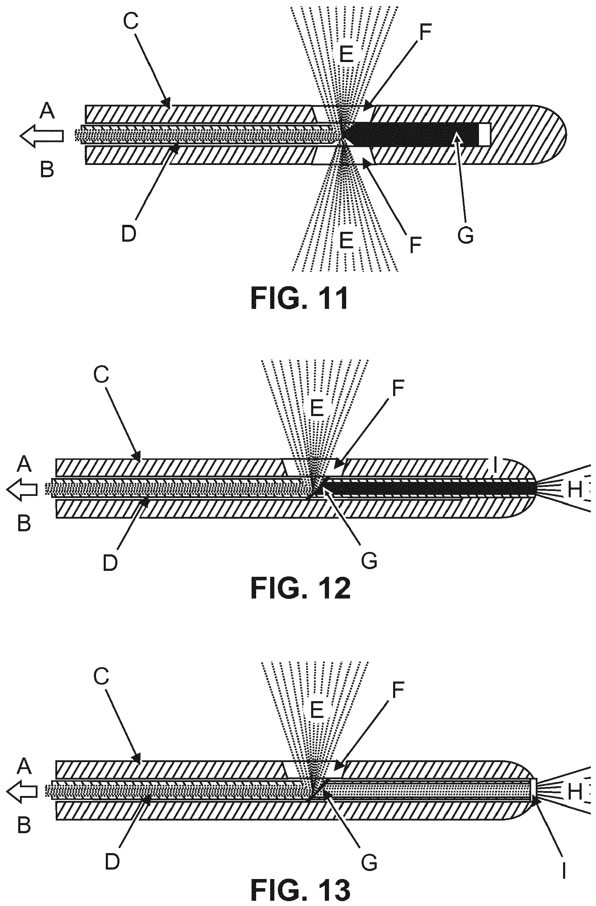

Fig. 11 shows an example of an intravascular guidewire. An "optical radiation source" is represented by "A", a "spectrometer" by "B", a "hollow tube" by "C", an "optical fiber" by "D", "emitted optical wavelength radiation" by "E", "holes or slit with transparent filing" by "F", "reflective cone" by "G", and a "tip" by "H". In this embodiment a cone shaped mirror is disposed adjacent a distal end of the fiber to generate the 360 degree ring of optical wavelength radiation around the wire (to generate an annulus or doughnut shape field of optical wavelength radiation). Ideally, the angle of the mirror cone is 45 degrees. There are also other, less efficient ways to shape the optical wavelength radiation profile, for example a scattering medium may be disposed in front of the optical fiber.

Fig. 12 shows an example of an intravascular guidewire. An "optical radiation source" is represented by "A", a "spectrometer" by "B", a "hollow tube" by "C", an "optical fiber" by "D", a "first sensing volume" by "E", "holes with transparent filling" by "F", "wavelength selective element" by "G", a "second sensing volume " by "H", and a "tip" by "I". While a side-sensing wire offers a benefit over a forward-sensing wire in many cases as discussed above, there are situations where a forward-sensing guidewire has benefits. This is especially the case if the physician does not want to pass the clot. It would therefore be highly beneficial if a single wire could offer both sensing option simultaneously or the physician could just switch between them. Thus, measurements at both locations simultaneously, because these regions can be probed with different wavelength ranges. However, it can be beneficial to e.g. limit the optical wavelength radiation exposure and provide actual switching. Thus, switching can indeed be achieved by just changing the spectrum of the optical wavelength radiation coupled into the fiber. Another way to offer both sensing options is to use two optical fibers in the wire, one for forward sensing and one for sideway sensing. But, this sideways and forwards sensing can be provided by one fiber. There can be therefore one sideways beam and one forwards beam, or two or more sideways beams (that could form a doughnut shape emission) and a forwards emission, and they can operate over the same wavelength range or over different wavelength ranges.

Thus, the system enables different sensing volumes to be interrogated by using different wavelengths or wavelengths ranges for each sensing volume. For example a wavelength range of approximately 450 nm to 900 nm may be used, this being predominantly visible optical radiation, to discriminate between different types of blood clots in one sensing volume, and a wavelength range of approximately 1000-1600 nm, this being near infrared, i.e. NIR, optical radiation, to simultaneously discriminate between different types of blood clots in a second sensing volume. The same, or similar wavelengths may alternatively be used, for example one could use wavelengths of 520 nm, 830 nm, 1270 nm, and 1450 nm to discriminate between different types of blood clot in one sensing volume while simultaneously using wavelengths of 525 nm, 835 nm, 1275 nm and 1455 nm to discriminate between different types of blood clots in a second sensing volume. Also, one can use wavelength selective elements integrated into the wire to ensure that sensing volume E is illuminated with optical wavelength radiation having different wavelengths to sensing volume H. Different types of wavelength-selective elements can be used. For example one could use a wavelength-selective element like a Fiber Bragg Grating, i.e. FBG, to couple optical wavelength radiation within a predetermined wavelength range out of the fiber. A depiction of this shown in Fig. 12.

Fig. 13 shows an example of an intravascular guidewire. A "optical radiation source" is represented by "A", a "spectrometer" by "B", a "hollow tube" by "C", an "optical fiber" by "D", a "sensing volume 1 (visible optical radiation)" by "E", a "visible transmitting IR absorbing window" by "F", a "50:50 splitter/coupler" by "G", a "sensing volume 2 (infrared optical radiation)" by "H", and a "visible absorbing IR transmitting window" by "I". In this embodiment about half of the optical wavelength radiation is coupled out at the first sensing volume without regard for wavelength and then optical filters are used to filter out the optical wavelength radiation one does not want to use. These optical filters may be integrated into the optical windows, or the optical filling, that seal an opening in the hollow tube. A 50:50 splitter/coupler has been referred to here, however other ratios can be utilized as would be appreciated by the skilled person.

Thus, in summary the above disclosure relates to an intravascular device, with an associated optical wavelength radiation generation and spectral data generation system. The intravascular device may have one or more of the following features: i) at least one optical fiber intended to emit optical wavelength radiation into biological tissue inside or adjacent to a blood vessel and to collect part of the optical wavelength radiation reflected by the tissue; ii) part of the optical wavelength radiation being emitted and received in a direction perpendicular to the main axis of the intravascular device; iii) part of the optical wavelength radiation emitted and received significantly proximally from the tip of the intravascular device (significantly proximally from the tip can mean more than 3 centimeters away from the tip, and if the intravascular device comprises a functional tip with a coil, proximally from the length covered by the coil; a coil being a standard design element in medical guidewires to achieve the desired mechanical properties wherein the tip of a guidewire typically has to be more flexible than the shaft; coils thus being used to provide the desired "floppiness"); iv) a tip that is optimized for its mechanical properties and not for its optical or sensing properties, v) emitting and receiving optical wavelength radiation from/to the tip, which can involve use of a wavelength-selective element, such that different optical wavelengths are emitted/received at different sensing volumes, vi) emitting and receiving optical wavelength radiation from/to a sideways direction, which can involve the use of a wavelength-selective element for emitting / receiving different optical wavelengths at different sensing volumes, vii) emitting and receiving optical wavelength radiation from/to a sideways direction in an annulus or doughnut shape, such that rotation or torqueing of the guidewire is not required in order to provide rotational sensitivity, viii) an intravascular microcatheter that is optically transparent at a predetermined length from the tip, with a lumen suitable to receive a guidewire, such that the intravascular device can measure from within the catheter through the catheter wall, ix) a (radiopaque) marker (or other feedback mechanism) that helps torqueing of the intravascular device to maximize the overlap between blood clot and the spectroscopic sensing volume.

An intravascular device for use in determining blood clot composition in the peripheral vasculature shares many of the features described above with reference to Fig. 1. By contrast however, it is not essential to include the aforementioned optical interaction element 40, or to provide the aforementioned two optical beams. With reference to Fig. 1, an intravascular device for determining blood clot composition in the peripheral vasculature may therefore include:

- an elongate member 20; and
- an optical fiber 30;

wherein at least a part of the elongate member 20 is configured to be inserted into a part of a vascular system of a patient;
wherein at least a part of the optical fiber 30 is located within the elongate member 20;
wherein, the optical fiber is configured to transmit optical wavelength radiation;
wherein the intravascular device is configured to emit a portion of the optical wavelength radiation out of the elongate member for being scattered and/or reflected by a portion of the vascular system; and
wherein the intravascular device is configured to collect at least some of the scattered and/or reflected optical wavelength radiation and to couple the at least some of the scattered and/or reflected optical wavelength radiation into the optical fiber.

In the device for use in determining blood clot composition in the peripheral vasculature, optionally:
In one example, the optical wavelength radiation is generated by an optical radiation source that emits broadband optical radiation simultaneously.

In one example, the optical wavelength radiation is generated by an optical radiation source that emits narrowband optical radiation and scans the emitted optical wavelengths over a wavelength range to generate broadband optical radiation that is transmitted by the optical fiber.

In another example, the intravascular device is a guidewire.

In another example, the intravascular device is a microcatheter.

In another example, the elongate member comprises a radio opaque marker.

In another example, the elongate member is a tube.

In another example, the optical fiber comprises a radio opaque marker.

In another example, the optical wavelength radiation may be emitted axially from an end wall of the elongate member, as illustrated with reference to Fig. 5.

In another example, the optical wavelength radiation may be emitted radially from the elongate member, for example in the form of a conical emission profile as may be provided by a beam redirector in the form of a region of the optical fiber exhibiting total internal reflection; or a beam redirector in the form of a mirror, or a beamsplitter, arranged transversally with respect to a longitudinal axis of optical fiber 30.

In another example, the optical wavelength radiation may be emitted radially from the elongate member in the form of an annular emission profile, for example by providing the optical fiber 30 with a beam redirector in the form of a distal tip having a conical shape as illustrated with reference to Fig. 10, or for example by providing a beam redirector in the form of a reflective cone as described with reference to Fig. 11.

In another example the radial emission may be in a direction perpendicular to a longitudinal axis of the elongate member.

In another example the radial emission may be provided at a position at least 3 centimeters from an end of the elongate member.

In another example, the intravascular device for use in determining blood clot composition in the peripheral vasculature may be incorporated into a microcatheter 110 as illustrated with reference to Fig. 2. The optical fiber 30 may be provided with a beam redirector as described above in order to emit optical wavelength radiation radially from the elongate member. At least a part of microcatheter 110 in Fig. 2 is configured to be inserted into the part of the vascular system of the patient. The microcatheter comprises at least one optically-transmitting wall portion configured to emit the optical wavelength radiation. The intravascular device is configured to slide within the microcatheter along a longitudinal axis of the microcatheter. The microcatheter and intravascular device are configured such that when the intravascular guidewire is positioned at one or more longitudinal positions along the longitudinal axis of the microcatheter, optical wavelength radiation is emitted out of the microcatheter through the at least one optically-transmitting wall portion of the microcatheter and scattered and/or reflected optical wavelength radiation enters the microcatheter through the at least one optically-transmitting wall portion of the microcatheter.

An intravascular investigation system 200 for use in determining blood clot composition in the peripheral vasculature is now described with reference to Fig. 3. The system comprises an intravascular device for use in determining blood clot composition in the peripheral vasculature as described above. The intravascular device for use in determining blood clot composition in the peripheral vasculature may optionally be included in a microcatheter as described above with reference to Fig. 2. System 200 for use in determining blood clot composition in the peripheral vasculature also comprises an optical radiation source 210, an optical radiation detector 220, and a processing unit 230. The optical radiation source is configured to generate optical wavelength radiation over a broadband range and couple it into the optical fiber. The optical radiation detector is configured to generate at least one detection signal on the basis of the scattered and/or reflected optical wavelength radiation. The processing unit is configured to determine at least one spectrally resolved data set on the basis of the at least one detection signal. The at least one spectrally resolved data set comprises a spectrum corresponding to collagen. The processing unit is configured to determine a collagen content from the spectrum corresponding to collagen and to determine information about a blood clot on the basis of the collagen content.

As described in more detail below, the presence of collagen in a peripheral vascular clot is a sign of advanced clot differentiation and age. In these clots, fibroblasts have had time to infiltrate the clot and to start the process of forming an endothelial layer covering the surface of the clot. In these chronic clots, t-PA and other thrombolytic agents may not be able to penetrate the thrombus depending on the degree of endothelialization. Therefore a blood clot with a significant collagen content may resist thrombolysis. In fact, attempting thrombolysis on these kind of clot may put the patient in danger, as the blood clot may become mobile and cause acute damage downstream, e.g. in the form of lung embolism. Therefore determining the collagen content of a peripheral vascular clot in this manner may be informative to a medical practitioner and allow them to choose the best mode of treatment for each patient.

Blood Clot Differentiation

The following provides details on the differentiation between a first blood clot type that is rich in red blood cells and a second type of blood clot that is rich in fibrin, which can comprise a determination of the amount of red blood cells present and/or the amount of fibrin present. In other words, graduations between on the one hand a "rich in red blood cell clot" and a "rich in fibrin clot" can take into account that real blood clots can exist between being a rich in red blood cell clot and a rich in fibrin clot, and could exist as a blood clot half way between the these two.

As discussed above the presently described system includes an intravascular device or microcatheter for optically interrogating a part of an intravascular system of a patient. Broadband optical wavelength radiation extending across a plurality of optical wavelengths may be used in the optical interrogation. Thus, a true broadband optical source may for example be used. Broadband optical wavelength radiation may also be provided in the form of a tunable laser, or in the form of a plurality of narrowband optical radiation sources such as LEDs or lasers emitting narrowband optical radiation simultaneously or sequentially. Thus, in an example the optical radiation source and detector can operate as a spectral resolving unit, where broadband optical wavelength radiation coupled into the optical fibre of the intravascular device involves the tuneable laser operating over a range of wavelengths and this optical wavelength radiation is scattered and/or reflected back from the patient and detected to provide a spectrally resolved data set. In another example, a one shot broadband optical wavelength radiation beam can be coupled into the fibre and collected and analyzed, for example by a spectrometer to provide the spectrally resolved data set.

The present described system enables the graduation in the actual form of the blood clot to be determined. The differentiation between a first blood clot type and a second blood clot type can comprise a determination of at least one physiological parameter, wherein the at least one physiological parameter comprises one or more of: amount of haemoglobin; haemoglobin oxygen saturation; an amount of scattering; a vessel packaging parameter; water content; and the amount of at least one haemoglobin derivative. The at least one physiological parameters can determined, for example, by fitting an optical model derived from diffusion theory to the measured spectra. In an example, fitting the spectra to an optical model can include taking into account a wavelength dependent absorption coefficient and a wavelength dependent reduced scattering coefficient. In an example, a double power law can be used to describe the wavelength dependence of the reduced scattering, where a first law corresponds to the contribution of Mie scattering and a second power law corresponds to the contribution of Rayleigh scattering. The reduced scattering $\mu_s'$ expressed in wavenumbers, i.e. cm$^{-1}$, as a function of wavelength $\lambda$ can be written as

$$\mu_s' = a\left(\rho_{MR}\left(\frac{\lambda}{\lambda_0}\right)^{-b} + (1 - \rho_{MR})\left(\frac{\lambda}{\lambda_0}\right)^{-4}\right)$$

Where, $\lambda_0$ is a normalization wavelength, that in an example can be set to 800nm, and the parameter *a* corresponds to the reduced scattering amplitude at this exemplar wavelength. The reduced scattering corresponds to the sum of Mie and Rayleigh scattering and $\rho_{MR}$ is defined as the Mie-to-Rayleigh fraction of the scattering. The reduced scattering slope of the Mie scattering is denoted b, and is related to the particle size.

More detail on the determination of the physiological parameters can be found in the following two papers: R. Nachabé, B. H. W. Hendriks, A. E. Desjardins, M. van der Voort, M. B. van der Mark, and H. J. C. M. Sterenborg, "Estimation of lipid and water concentrations in scattering media with diffuse optical spectroscopy from 900 to 1600 nm", J. Biomed. Opt. 15, (2010); and Rami Nachabé, Benno H. W. Hendriks, Marjolein van der Voort, Adrien E. Desjardins, and Henricus J. C. M. Sterenborg, "Estimation of biological chromophores using diffuse optical spectroscopy: benefit of extending the UV-VIS wavelength range to include 1000 to 1600 nm", Optics Express 18 (2010) p1432.

The determination of the at least one physiological parameter can comprises one or more of: a fitting of an optical model to the at least one spectrally resolved data set; application of at least one multivariate analysis tool to the at least one spectrally resolved data set; a partial least squares discriminant analysis of the at least one spectrally resolved data set; application of support vector machines to the at least one spectrally resolved data set; application of a k nearest neighbor analysis; and application of deep learning algorithms to the at least one spectrally resolved data set. The at least one multivariate analysis tool comprises principle component analysis, PCA. The differentiation between the first blood clot type and the second blood clot type can comprise utilization of a look-up-table.

In the above-described intravascular investigation system 200 for use in determining blood clot composition in the peripheral vasculature, a similar optical analysis technique may be used to determine a collagen content. In the system 200, a spectrally resolved data set is generated. The spectrally resolved data set comprises a spectrum corresponding to collagen. The processing unit is configured to determine a collagen content from the spectrum corresponding to collagen and to determine information about a blood clot on the basis of the collagen content.

In peripheral vascular disease it has been found that a discrimination between recently-formed (i.e. acute) and older (i.e. chronic) blood clots can be made on the basis of the collagen content of the clot. This is because the collagen content increases as the clot ages. The age of the clot is a useful factor for use by a medical practitioner to use to determine which of multiple treatment options are most suitable for the clot, for example whether to perform a thrombectomy or a thrombolysis, and consequently which of the various treatment devices to use. In one example implementation the collagen content can be determined by fitting the aforementioned optical model to the measured spectra, as described for a diffuse reflectance spectroscopy technique with reference to Equation 4 and Fig. 2 of Nachabé et al. 2011. Collagen exhibits absorption bands in the wavelength range 400 - 1700 nm which is particularly well-suited to the use of readily-available optical components. Particularly useful absorption bands that are characteristic of Collagen occur at approximately 950 nm $\pm$ 50nm, 1030 nm $\pm$ 50nm, 1230 nm $\pm$ 50 nm and 1500 nm $\pm$ 100 nm.

In order to demonstrate that the Collagen content could be accurately determined using the Diffuse Reflectance Spectroscopy technique, various blood clot "analogue" samples were prepared from Collagen and Fibrin and their spectra were measured using a spectrophotometer. Collagen in the analogue sample is representative of the Collagen in the blood clot, and Fibrin was added as the counterpart of the blood clot analogue samples in order represent the Fibrin that is typically also present in a real blood clot, and also to demonstrate that Collagen could be measured in the presence of the relatively close spectral signature characteristics of Fibrin.

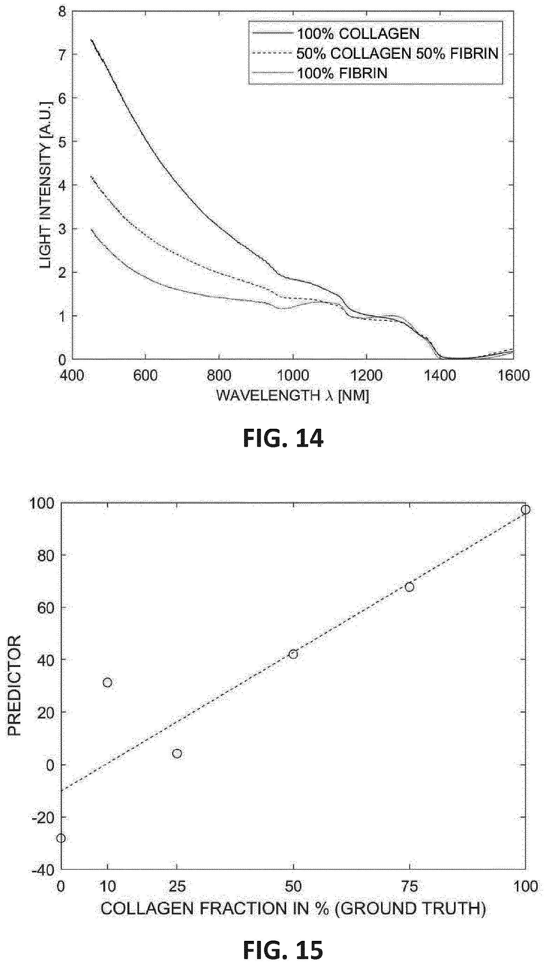

Thereto, Fig. 14 illustrates the variation in measured light intensity (arbitrary units) versus wavelength in nanometers for three blood clot analogue samples. In the uppermost graph in Fig. 14 the spectrum of a pure Collagen sample (100 % Collagen) was measured. In the lowermost graph in Fig. 14 the spectrum of a pure Fibrin sample (100 % Fibrin) was measured. In the central graph there was 50 % Collagen and 50 % Fibrin. The characteristic absorption bands of Collagen occur can be seen in Fig. 14 at approximately 950+/- 50nm, 1030nm+/-50nm, 1230 nm +/- 50 nm and 1500 nm +/- 100 nm.

The collagen content "Predictor" was subsequently determined using the aforementioned optical model for the spectra of Fig. 14, and also for spectra of analogue samples having a collagen fraction of 25 % and 75 %, and compared against the ground truth collagen fraction, as illustrated in Fig. 15. Fig. 15 illustrates the predictive ability (Predictor) versus Collagen fraction (%), i.e. the ground truth, for several measured blood clot analogue samples. Ideally the value of the parameter Predictor in Fig. 15 would be equal to the Collagen Fraction in Fig. 15. As can be seen from Fig. 15, the model accuracy improves with Collagen fraction.

Various alternative optical arrangements are also suitable for the measurement of collagen content including Raman spectroscopy which is a molecular specific technique with unique spectral features useful for the detection of collagen.

A corresponding method of determining the collagen content of a peripheral vascular clot with the aforementioned intravascular investigation system 200 for use in determining blood clot composition in the peripheral vasculature may include the steps of:

- generating optical wavelength radiation over a broadband range with the optical radiation source;
- coupling the broadband optical wavelength radiation into the optical fiber of the intravascular device or the intravascular microcatheter and guidewire device;
- collecting scattered and/or reflected optical wavelength radiation from a vascular structure of the patient with the intravascular device or the intravascular microcatheter and guidewire device;
- generating by the optical wavelength radiation detector at least one detection signal on the basis of the scattered and/or reflected optical wavelength radiation;
- determining by the processing unit at least one spectrally resolved data set on the basis of the at least one detection signal, the at least one spectrally resolved data set comprising a spectrum corresponding to collagen; and
- determining by the processing unit a collagen content from the spectrum corresponding to collagen, and information about a blood clot on the basis of the collagen content.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments. The computer program or the output unit may be integrated into an imaging or a navigation system.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items recited in the claims. The mere fact that certain measures are re-cited in mutually different dependent claims does not indicate that a combination of these measures cannot be used to advantage. Any reference signs in the claims should not be construed as limiting the scope.

## Claims

1. An intravascular device (10), comprising:

   - an elongate member (20);
   - an optical fiber (30); and
   - at least one optical interaction element (40);

   wherein at least a part of the elongate member (20) is configured to be inserted into a part of a vascular system of a patient;
   wherein at least a part of the optical fiber (30) is located within the elongate member (20);
   wherein, the optical fiber is configured to transmit optical wavelength radiation;
   wherein the intravascular device is configured to emit a portion of the optical wavelength radiation out of the elongate member in at least two optical radiation beams for being scattered and/or reflected by a portion of the vascular system, wherein the emission of the at least two optical radiation beams comprises interaction of the transmitted optical wavelength radiation with the at least one optical interaction element; and
   wherein the intravascular device is configured to collect at least some of the scattered and/or reflected optical wavelength radiation and to couple the at least some of the scattered and/or reflected optical wavelength radiation into the optical fiber comprising utilization of the at least one optical interaction element.

2. Device according to claim 1, wherein the at least two optical radiation beams comprises a first optical radiation beam emitted out of a side wall of the elongate member.

3. Device according to claim 2, wherein the at least two optical radiation beams comprises a second optical radiation beam emitted out of the side wall of the elongate member.

4. Device according to claim 3, wherein a wavelength range of the first optical radiation beam is different to a wavelength range of the second optical radiation beam.

5. Device according to any of claims 3-4, wherein the first optical radiation beam is emitted out of the elongate member at a first longitudinal position of the elongate member and the second optical radiation beam is emitted out of the elongate member at a second longitudinal position of the elongate member different to the first longitudinal position.

6. Device according to any of claims 2-5, wherein the at least two optical radiation beams comprises an optical radiation beam emitted out of an end wall of the elongate member.

7. Device according to claim 6, wherein a wavelength range of the first optical radiation beam is different to a wavelength range of the optical radiation beam emitted out of the end wall of the elongate member.

8. Device according to claim 7 when claim 6 is dependent upon any of claims 3-5, wherein a wavelength range of the second optical radiation beam is different to the wavelength range of the optical radiation beam emitted out of the end of the elongate member.

9. Device according to any of claims 1-8, wherein the at least one optical interaction element comprises a wavelength selective element.

10. Device according to any of claims 1-10, wherein a portion of the optical fiber at a distal end of the optical fiber is fixedly connected to the elongate member; and wherein the at a least a part of the optical fiber located within the elongate member other than the fixed distal end is not fixedly connected to the elongate member.

11. An intravascular device (10), comprising:

   - an elongate member (20);
   - an optical fiber (30); and
   - at least one optical interaction element (40);

   wherein at least a part of the elongate member is configured to be inserted into a part of a vascular system of a patient;
   wherein at least a part of the optical fiber is located within the elongate member;
   wherein the optical fiber is configured to transmit optical wavelength radiation;
   wherein the intravascular device is configured to emit at least a portion of the optical wavelength radiation out of the elongate member in an optical radiation beam that forms an annular emission profile substantially perpendicular to a longitudinal axis of the elongate member for being scattered and/or reflected by a portion of the vascular system, wherein the emission of the optical radiation beam comprises interaction of the transmitted optical wavelength radiation with the at least one optical interaction element; and
   wherein the intravascular device is configured to collect at least some of the scattered and/or reflected optical wavelength radiation and couple the at least some of the scattered and/or reflected optical wavelength radiation into the optical fiber comprising utilization of the at least one optical interaction element.

12. An intravascular microcatheter and guidewire device (100), comprising:

   - a microcatheter (110); and
   - an intravascular device (10) according to any of claims 1-10 or an intravascular device (100) according to claim 11;

   wherein at least a part of the microcatheter is configured to be inserted into the part of the vascular system of the patient;

wherein the microcatheter comprises at least one optically-transmitting wall portion;
wherein the intravascular device is configured to slide within the microcatheter along a longitudinal axis of the microcatheter; and
wherein the microcatheter and intravascular device are configured such that when the intravascular guidewire is positioned at one or more longitudinal positions along the longitudinal axis of the microcatheter, optical wavelength radiation is emitted out of the microcatheter through the at least one optically-transmitting wall portion of the microcatheter and scattered and/or reflected optical wavelength radiation enters the microcatheter through the at least one optically-transmitting wall portion of the microcatheter.

13. An intravascular investigation system (200), comprising:

   - an intravascular device (10) according to any of claims 1-10, or an intravascular device (10) according to claim 11, or an intravascular microcatheter and guidewire device (100) according to claim 12;
   - a optical radiation source (210);
   - a optical radiation detector (220); and
   - a processing unit (230);

   wherein, the optical radiation source is configured to generate optical wavelength radiation over a broadband range and couple it into the optical fiber;
   wherein, the optical radiation detector is configured to generate at least one detection signal on the basis of the scattered and/or reflected optical wavelength radiation;
   wherein, the processing unit is configured to determine at least one spectrally resolved data set on the basis of the at least one detection signal; and
   wherein, the processing unit is configured to determine information about a blood clot on the basis of the at least one spectrally resolved data set.

14. A method of intravascular investigation with an intravascular investigation system (200) according to claim 13, wherein the method comprises:

   - generating optical wavelength radiation over a broadband range with the optical radiation source;
   - coupling the broadband optical wavelength radiation into the optical fiber of the intravascular device or the intravascular microcatheter and guidewire device;
   - collecting scattered and/or reflected optical wavelength radiation from a vascular structure of the patient with the intravascular device or the intravascular microcatheter and guidewire device;
- generating by the optical wavelength radiation detector at least one detection signal on the basis of the scattered and/or reflected optical wavelength radiation;
- determining by the processing unit at least one spectrally resolved data set on the basis of the at least one detection signal; and
- determining by the processing unit information about a blood clot on the basis of the at least one spectrally resolved data set.

15. A computer program element for controlling a system according to claim 13, which when executed by a processor is configured to carry out the method of claim 14.

**FIG. 1**

**FIG. 2**

**FIG. 3**

A    B    C    D

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

**FIG. 15**

Europäisches
Patentamt

European
Patent Office

Office européen
des brevets

## EUROPEAN SEARCH REPORT

Application Number

EP 19 20 5692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/137949 A1 (KONINKLIJKE PHILIPS NV [NL]) 2 August 2018 (2018-08-02) * the whole document * ----- | 1-15 | INV. A61B5/00 A61B5/02 |
| Y | WO 2016/205576 A1 (CORNING INC [US]) 22 December 2016 (2016-12-22) * paragraph [0050] - paragraph [0051] * * figures 4c, 4d * ----- | 1-10, 12-15 | |
| Y | US 6 016 440 A (SIMON ARNO [DE] ET AL) 18 January 2000 (2000-01-18) * column 6, line 31 - line 40 * * column 6, line 60 - column 7, line 7 * * figures 2, 4 * ----- | 11 | |

TECHNICAL FIELDS
SEARCHED (IPC)

A61B

The present search report has been drawn up for all claims

| Place of search | Date of completion of the search | Examiner |
|---|---|---|
| The Hague | 11 March 2020 | Van Dop, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 20 5692

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2020

| Patent document cited in search report | | Publication date | Patent family member(s) | | Publication date |
|---|---|---|---|---|---|
| WO 2018137949 | A1 | 02-08-2018 | EP | 3573514 A1 | 04-12-2019 |
| | | | JP | 2020505979 A | 27-02-2020 |
| | | | US | 2019365248 A1 | 05-12-2019 |
| | | | WO | 2018137949 A1 | 02-08-2018 |
| WO 2016205576 | A1 | 22-12-2016 | US | 2018364024 A1 | 20-12-2018 |
| | | | WO | 2016205576 A1 | 22-12-2016 |
| US 6016440 | A | 18-01-2000 | DE | 19732215 A1 | 05-02-1998 |
| | | | GB | 2315862 A | 11-02-1998 |
| | | | US | 6016440 A | 18-01-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5439000 A
- US 5601087 A
- US 7532920 B1
- US 6445939 B1

### Non-patent literature cited in the description

- **T. ANDERSSON.** *The importance of clot properties in endovascular stroke therapy,* 2015, https://neuronewsinternational.com/the-importance-of-clot-properties-in-endovascular-stroke-therapy/
- **R. NACHABÉ ; B. H. W. HENDRIKS ; A. E. DESJARDINS ; M. VAN DER VOORT ; M. B. VAN DER MARK ; H. J. C. M. STERENBORG.** Estimation of lipid and water concentrations in scattering media with diffuse optical spectroscopy from 900 to 1600 nm. *J. Biomed. Opt.,* 2010, vol. 15
- **RAMI NACHABÉ ; BENNO H. W. HENDRIKS ; MARJOLEIN VAN DER VOORT ; ADRIEN E. DESJARDINS ; HENRICUS J. C. M. STERENBORG.** Estimation of biological chromophores using diffuse optical spectroscopy: benefit of extending the UV-VIS wavelength range to include 1000 to 1600 nm. *Optics Express,* 2010, vol. 18, 1432
- **R. NACHABÉ et al.** Diagnosis of breast cancer using diffuse optical spectroscopy from 500 to 1600 nm: comparison of classification methods. *J. Biomed. Opt.,* 2011, vol. 16 (8), 087010